# FASCICULE DE BREVET EUROPEEN

(11) **EP 3 471 788 B1**
(45) Date de publication et mention de la délivrance du brevet: **02.09.2020**
(21) Numéro de dépôt: 17734262.3
(22) Date de dépôt: 16.06.2017
(51) Int. Cl.: A61L 27/36

(54) **PROTHESE BIOLOGIQUE DESTINEE AU TRAITEMENT DES HERNIES PARASTOMALES**
BIOLOGISCHE PROTHESE ZUR BEHANDLUNG PARASTOMALEN HERNIEN
BIOLOGICAL PROSTHESIS INTENDED FOR THE TREATMENT OF PARASTOMAL HERNIAS

(30) Priorité: 17.06.2016 FR 1655645
(43) Date de publication de la demande: 24.04.2019
(73) Titulaire: Meccellis Biotech, 17000 La Rochelle (FR)
(72) Inventeur: PERES, Anthony, 17139 Dompierre-sur-Mer (FR); HOFMANSKI, Guillaume, 61250 Mieuxce (FR); LAMMELIN, Aurélie, 78550 Richebourg (FR)
(74) Mandataire: Aquinov
(86) Numéro de dépôt international: PCT/EP2017/064793
(87) Numéro de publication internationale: WO 2017/216357

(56) Documents cités:
- US-A1- 2013 209 572
- US-A1- 2016 030 487

## Description

La présente invention se rapporte à une nouvelle prothèse biologique fabriquée à partir d'une queue animale, utile en particulier dans le traitement des hernies parastomales.

Une stomie est une petite ouverture, temporaire ou permanente, créée lors d'une opération chirurgicale pour évacuer les selles lorsqu'elles ne peuvent plus l'être par les voies naturelles. On sait que 50 à 70% des patients qui ont une stomie définitive ont une hernie parastomale dans les trois premières années. Les patients doivent alors subir une nouvelle intervention pour traiter la hernie. Or, une telle intervention est compliquée et le taux de récidive est élevé. Il est donc nécessaire d'associer un matériel prothétique à la chirurgie.

Actuellement, les prothèses utilisées sont des prothèses synthétiques. Or, un matériel prothétique synthétique est déconseillé dans ce type de chirurgie car il provoque de l'inconfort chez le patient et des douleurs. En outre, et surtout, l'utilisation d'un implant synthétique n'est pas recommandé dans le cadre de chirurgies septiques ou potentiellement septiques comme c'est le cas des interventions en chirurgie parastomale. Si une infection se produit, il faut absolument retirer la prothèse car elle augmente les risques de complications.

L'objectif de l'invention est de résoudre ces problématiques et de proposer une prothèse biologique à la fois adaptée aux chirurgies septiques, évitant les douleurs, répondant aux exigences du traitement des hernies parastomales et simple de fabrication et d'utilisation. A cet effet, la présente invention propose d'utiliser du derme prélevé sur une partie spécifique des animaux, située au niveau de la queue.

La peau animale, en particulier la peau de porcins, est déjà utilisée depuis plusieurs années pour la fabrication de prothèses biologiques en chirurgie de la paroi. US2013/209572 décrit une prothèse biologique constituée de derme prélevé sur des parties caudales du porc pour le traitement des hernies. Néanmoins, l'utilisation du derme animal pour la fabrication de prothèse dans le cas de la hernie parastomale nécessiterait de nombreuses manipulations et assemblages de parties de dermes pour obtenir la forme désirée, ce qui semble difficilement réalisable à la fois d'un point de vue technique et d'un point de vue économique. Toutes références à des méthodes de traitement ou des méthodes chirurgicales font références à la prothèse de la présente invention pour utilisation dans lesdites méthodes.

La présente invention vise une prothèse biologique fabriquée à partir d'une partie très particulière de l'animal, ce qui évite ces problématiques.

En effet, l'invention vise une prothèse biologique selon les revendications 1 à 11 présentant une épaisseur comprise entre 0,5 et 4 mm, constituée par du derme prélevé sur une partie d'un animal appartenant à la famille des porcins, des bovins ou des caprins, ladite partie de l'animal correspondant à une partie allant de la zone située à la base de la queue en regard de la jonction des dernières vertèbres sacrées jusqu'à une zone partant de la base de la queue recouvrant la circonférence d'au moins une partie des vertèbres caudales.

Avantageusement, une telle prothèse possède une forme tout à fait adaptée au traitement des hernies parastomales. Elle est simple de fabrication, et s'agissant d'une prothèse biologique, elle est adaptée à l'intervention chirurgicale qui doit être réalisée dans des conditions septiques ou potentiellement septiques, et enfin elle ne crée pas de douleurs aux patients contrairement aux prothèses pour hernie parastomale, existantes. D'autres caractéristiques et avantages ressortiront de la description en détail de l'invention qui va suivre en regard des figures annexées sur lesquelles :
- La figure 1 représente un schéma de la prothèse selon l'invention,
- La figure 2 représente un schéma du prélèvement de la partie de l'animal pour fabriquer la prothèse selon l'invention, et
- La figure 3 représente une photographie d'une prothèse selon l'invention.

### DEFINITIONS

Par « acellularisation » au sens de l'invention on entend l'élimination des éléments cellulaires. Pour que la prothèse puisse être implantée chez un receveur, les tissus prélevés sur l'animal donneur sont décellularisés de façon à diminuer leurs immunogénécités. Ce procédé implique l'élimination des cellules de l'animal donneur tout en conservant les qualités biologiques et mécaniques de la matrice extracellulaire.

Par « déantigénisation » au sens de l'invention on entend l'élimination de protéines présentes dans le tissu de l'animal donneur qui pourraient ne pas être reconnues par le receveur de la prothèse provoquant ainsi le rejet de l'implant. En effet, l'animal donneur peut exprimer des enzymes ou protéines dans le tissu qui ne sont pas exprimées par le receveur. Il est par conséquent important d'éliminer ces éléments, comme notamment l'épitope galactose alpha-1,3 galactose (alpha-gal) présent chez l'espèce porcine, afin de diminuer la réponse immunitaire après implantation chez l'homme.

Par « tronc de cône » au sens de l'invention on entend la forme générale en forme de cône, ouvert à sa pointe, les bords, notamment du côté grand diamètre, pouvant être festonnés du fait de l'élasticité générale du matériau naturel. Le tronc de cône se situe à la base de la queue ; la base s'ouvre sur la partie dorsale de l'animal pour donner naissance à une collerette se situant perpendiculairement à la queue.

Par « de forme cylindrique » au sens de l'invention on entend une forme générale de révolution, pas nécessairement une forme cylindrique parfaite du fait du matériau naturel.

### DESCRIPTION DETAILLEE DE L'INVENTION

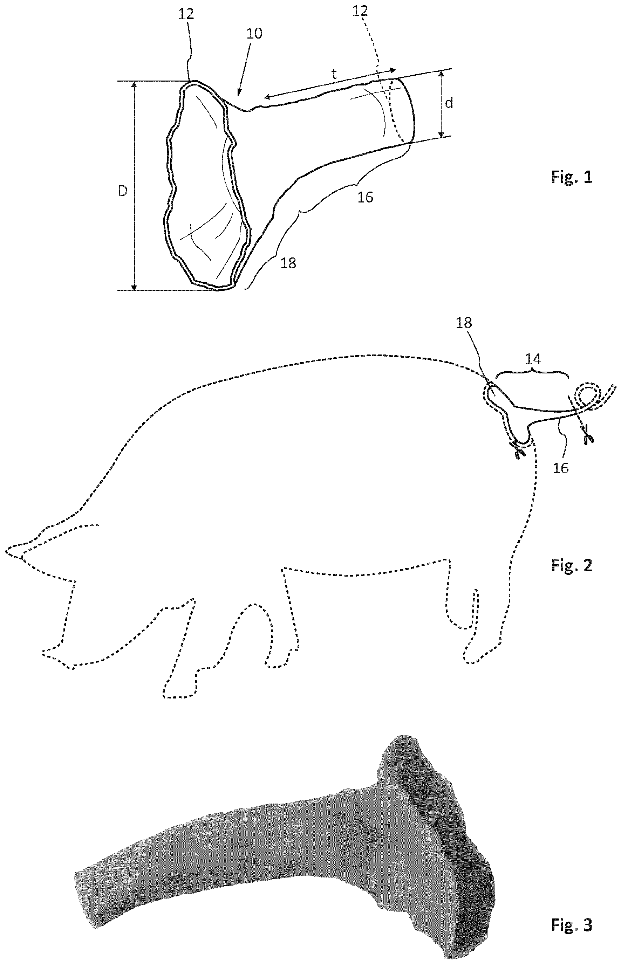

Selon un premier aspect, l'invention vise donc une prothèse biologique 10 telle que représentée sur la figure 1. Elle présente une épaisseur 12 comprise entre 0,5 et 4 mm, constituée par du derme prélevé sur une partie 14 d'un animal appartenant à la famille des porcins, des bovins ou des caprins, ladite partie 14 de l'animal correspondant à une partie allant de la zone 16 située à la base de la queue en regard de la jonction des dernières vertèbres sacrées jusqu'à une zone 18 partant de la base de la queue recouvrant la circonférence d'au moins une partie des vertèbres caudales.

Le derme animal, et en particulier le derme porcin, est particulièrement adapté car sa résistance mécanique permet son utilisation en chirurgie. En outre, la composition de la matrice extracellulaire, en particulier le collagène, du derme porcin est similaire à celle de l'intestin des êtres humains.

Pour améliorer la compatibilité, le derme est préférentiellement traité, en particulier par acellularisation et déantigénisation. Ce traitement permet d'éliminer notamment les éléments cellulaires et l'épitope alpha-gal qui peuvent provoquer une réaction immunitaire accrue et un rejet aigu de l'implant d'origine animale.

Plusieurs méthodes connues de l'homme du métier existent pour traiter le derme par acellularisation et déantigénisation, comme par exemple celles décrites dans :
- Badylak et al., "Extracellular Matrix as a Biological Scaffold Material: Structure and Function," Acta Biomaterialia (2008), doi:10.1016/j.actbio.2008.09.013
- Xu et al., Tissue Engineering, Vol. 15, 1-13 (2009).

L'épaisseur 12 du derme constituant la prothèse est comprise entre 0,5 et 4 mm, préférentiellement entre 0,5 et 2 mm. Cette épaisseur est nécessaire pour pouvoir assurer une bonne résistance mécanique à l'implant. L'épaisseur 12 varie en fonction de la résistance mécanique souhaitée, car plus l'épaisseur est importante, moins la matière est souple. Pour obtenir une telle épaisseur, il peut être nécessaire de fendre le derme dans l'épaisseur, à la dimension souhaitée, à l'aide de moyens tranchants comme un couteau par exemple.

La résistance mécanique de la prothèse biologique 10 selon l'invention répond préférentiellement aux paramètres suivants (mesurés selon les méthodes décrites dans Deken et al. « Physicochemical évaluation of absorbale and non absorbale barrier composite mesh for laparoscopic ventral hernia repair ») :
- Une force de rétention de suture (« Suture rétention strength ») supérieure à 20 N,
- Une résistance au déchirement (« Tear résistance ») supérieure à 20 N,
- Une force d'éclatement (« Ball burst strength ») supérieure à 50 N/cm.

Selon un mode de réalisation particulièrement adapté, la zone 16 de la prothèse, située à la base de la queue en regard de la jonction des dernières vertèbres sacrées est en forme de tronc de cône. Préférentiellement, la partie inférieure du tronc de cône a un diamètre D compris entre 2 et 8 cm, encore plus préférentiellement entre 4 et 7 cm.

La zone 18 correspond à tout ou partie de la queue de l'animal. Cette zone 18 est donc de forme cylindrique. Le diamètre moyen d de cette zone 18 est préférentiellement compris entre 1 et 4 cm, encore plus préférentiellement entre 1,5 et 3 cm. Ce diamètre est idéal pour une utilisation en chirurgie digestive pour le traitement de la hernie parastomale. Il peut être adapté en fonction du diamètre de la stomie.

Par ailleurs, la taille t de la zone 18 peut correspondre à la longueur de la queue ou à une longueur inférieure. Préférentiellement, cette taille t est comprise entre 1 et 8 cm, préférentiellement entre 1 et 4 cm.

La prothèse biologique 10 selon l'invention, pour être conservée, peut être séchée. Préférentiellement, elle est lyophilisée car cette technique de séchage est particulièrement adaptée à la préservation du collagène qui est une protéine très sensible à la température. Pour son utilisation la prothèse doit être réhydratée par tout procédé connu à cet effet.

La prothèse biologique 10 selon l'invention est fabriquée par un procédé de fabrication selon les revendications 12-13 comprenant les étapes suivantes :
- prélever le tégument de l'animal sur la partie 14 aux dimensions souhaitées,
- séparer le derme du reste du tégument,
- éventuellement fendre le derme prélevé dans l'épaisseur, pour obtenir l'épaisseur 12 souhaitée,
- traitement du derme, en particulier par acellularisation et déantigénisation,
- stérilisation.

Avant prélèvement, il est nécessaire de vérifier que la queue de l'animal a un diamètre d adapté à celui souhaité pour la prothèse. Cette mesure peut être réalisée par tout moyen, par exemple à l'aide d'un pied à coulisse.

Le prélèvement se fait sur un animal mort, juste après qu'il ait été abattu, directement en abattoir.

La séparation du derme du reste du tégument est réalisée à l'aide de tout moyen tranchant, comme un couteau par exemple, préférentiellement directement après le prélèvement en abattoir.

Le derme est fendu à l'aide de tout moyen tranchant, comme un couteau par exemple. Cette étape doit être réalisée avec précision de façon à obtenir une épaisseur uniforme sur l'ensemble de l'implant.

Avant ou après stérilisation, le procédé peut comprendre une étape de lyophilisation.

La prothèse biologique 10 selon l'invention est préférentiellement conditionnée dans un emballage stérile.

Avantageusement la prothèse biologique conserve les qualités de la matrice extracellulaire dont elle est extraite et favorise l'intégration tissulaire évitant ainsi l'érosion des tissus adjacents. En cas de sepsis post opératoire et/ou de récidive, la prothèse selon l'invention ne doit pas être retirée contrairement aux prothèses synthétiques.

Sa forme et ses dimensions sont parfaitement adaptées à une utilisation en chirurgie digestive pour le traitement des hernies parastomales.

L'invention a donc également pour objet une prothèse biologique 10 pour une utilisation en chirurgie digestive, en particulier dans le traitement des hernies parastomales.

La prothèse est utilisée comme les prothèses synthétiques existantes, préférentiellement par la mise en œuvre des étapes suivantes :
- Incision circulaire de la peau au niveau du muscle grand droit
- Incision de l'aponévrose antérieure du muscle grand droit
- Dissection circulaire au doigt rétro-musculaire
- Incision de l'aponévrose postérieur et du péritoine en fonction du diamètre (d) de la prothèse,
- Positionnement et fixation par des points séparés (6/8 sutures, résorption lente 2/0) sur l'aponévrose postérieure de la prothèse (partie 18)
- Fixation par des points séparés (4/6 sutures résorption lente) sur l'aponévrose antérieure de la prothèse (partie 16)
- Passage de l'anse digestive dans la prothèse
- Gestion de la stomie de manière conventionnelle.

L'invention est à présent illustrée par un exemple précis de prélèvement et d'obtention et des exemples de prothèses biologiques selon l'invention, obtenues selon ce procédé.

### Exemple de procédé

La queue de l'animal est prélevée selon les revendications après abattage et avant tout autre procédé de traitement.

Le derme de la queue est ensuite séparé avec un couteau des autres éléments anatomiques de la queue de façon à conserver au maximum l'étui cutané.

Afin d'obtenir l'épaisseur souhaitée, le derme peut être refendu à l'aide d'un couteau ou autres outils tranchants sur l'ensemble de la surface et de manière uniforme.

La prothèse ainsi obtenue est alors traitée par différents traitements chimiques de façon à éliminer les éléments cellulaires et autres molécules propres à l'organisme du donneur. La prothèse est ensuite lyophilisée pour une conservation optimale mais peut aussi être conservée par imprégnation d'une solution chimique adéquate.

Une ultime découpe est réalisée afin d'obtenir les dimensions finales souhaitées. Les implants sont ensuite stérilisés par les techniques conventionnelles (ETO, rayons gamma, Ebeam).

Exemples de prothèses obtenus selon ce procédé avec une épaisseur de 1,5 mm :

### Exemple 1 :

- t = 3 cm ; d = 2,5 cm ; D=4,5 cm

### Exemple 2 :

- t = 3 cm ; d = 3, 5 cm ; D = 5 cm

### Exemple 3 :

- t= 4 cm ; d = 4,5 cm ; D = 5 cm

## Revendications

1. Prothèse biologique (10) présentant une épaisseur (12) comprise entre 0,5 et 4 mm, constituée par du derme prélevé sur une partie (14) d'un animal appartenant à la famille des porcins, des bovins ou des caprins, ladite partie (14) de l'animal correspondant à une partie allant de la zone (16) située à la base de la queue en regard de la jonction des dernières vertèbres sacrées jusqu'à une zone (18) partant de la base de la queue recouvrant la circonférence d'au moins une partie des vertèbres caudales.

2. Prothèse biologique selon la revendication 1, **caractérisée en ce que** le derme est un derme traité par acellularisation et antigénisation.

3. Prothèse biologique (10) selon l'une des précédentes revendications, **caractérisée en ce que** la zone (16) située à la base de la queue en regard de la jonction des dernières vertèbres sacrées est en forme de tronc de cône.

4. Prothèse biologique (10) selon l'une des précédentes revendications, **caractérisée en ce que** la zone (18) recouvrant la circonférence d'au moins une partie des vertèbres caudales est de forme cylindrique.

5. Prothèse biologique (10) selon l'une des précédentes revendications, **caractérisée en ce que** le diamètre moyen de la zone (18) est compris entre 1 et 4 cm.

6. Prothèse biologique (10) selon l'une des précédentes revendications, **caractérisée en ce que** le diamètre (d) moyen de la zone (18) est compris entre 1,5 et 3 cm.

7. Prothèse biologique (10) selon l'une des précédentes revendications, **caractérisée en ce que** l'épaisseur (12) est comprise entre 0,5 et 2 mm.

8. Prothèse biologique (10) selon l'une des précédentes revendications, **caractérisée en ce que** la taille (t) de la zone (18) est comprise entre 1 et 8 cm.

9. Prothèse biologique (10) selon l'une des précédentes revendications, **caractérisée en ce que** la taille (t) de la zone (18) est comprise entre 1 et 4 cm.

10. Prothèse biologique (10) selon l'une des précédentes revendications, **caractérisée en ce que** la partie inférieure du tronc de cône a un diamètre D compris entre 2 et 8 cm.

11. Prothèse biologique (10) selon l'une des précédentes revendications **caractérisée en ce qu'**elle est lyophilisée.

12. Procédé de fabrication d'une prothèse biologique (10) selon l'une des précédentes revendications, **caractérisé en ce qu'**il comprend les étapes suivantes :
- prélever le tégument de l'animal sur la partie (14) aux dimensions souhaitées, le prélèvement se fait sur un animal mort, juste après qu'il ait été abattu, directement en abattoir,
- séparer le derme du reste du tégument,
- éventuellement fendre le derme prélevé dans l'épaisseur, pour obtenir l'épaisseur (12) souhaitée,
- traitement du derme par acellularisation et antigénisation,
- stérilisation.

13. Procédé de fabrication selon la précédente revendication, **caractérisé en ce qu'**il comprend, après traitement, une étape de lyophilisation et éventuellement de conditionnement dans un emballage stérile.

## Patentansprüche

1. Biologische Prothese (10) mit einer Dicke (12) zwischen 0,5 und 4 mm, die aus Dermis gebildet ist, die einem Teil (14) eines Tieres entnommen worden ist, das zu der Familie der Schweine, Rinder oder Ziegen gehört, wobei der Teil (14) des Tieres einem Teil entspricht, der sich von dem am Schwanzansatz gelegenen Bereich (16) gegenüber der Verbindung der letzten Kreuzbeinwirbel bis zu einem Bereich (18) erstreckt, der vom Schwanzansatz ausgeht und den Umfang wenigstens eines Teils der Schwanzwirbel abdeckt.

2. Biologische Prothese (10) nach Anspruch 1, **dadurch gekennzeichnet, dass** die Dermis eine durch Dezellularisierung und Antigenisierung behandelte Dermis ist.

3. Biologische Prothese (10) nach einem der vorhergehenden Ansprüche, **dadurch gekennzeichnet, dass** der im Bereich des Schwanzansatzes gegenüber der Verbindung der letzten Kreuzbeinwirbel gelegene Bereich als Kegelstumpf ausgebildet ist.

4. Biologische Prothese (10) nach einem der vorhergehenden Ansprüche, **dadurch gekennzeichnet, dass** der den Umfang wenigstens eines Teils der Schwanzwirbel abdeckende Bereich (18) zylinderförmig ausgebildet ist.

5. Biologische Prothese (10) nach einem der vorhergehenden Ansprüche, **dadurch gekennzeichnet, dass** der mittlere Durchmesser des Bereichs (18) zwischen 1 und 4 cm liegt.

6. Biologische Prothese (10) nach einem der vorhergehenden Ansprüche, **dadurch gekennzeichnet, dass** der mittlere Durchmesser (d) des Bereichs (18) zwischen 1,5 und 3 cm liegt.

7. Biologische Prothese (10) nach einem der vorhergehenden Ansprüche, **dadurch gekennzeichnet, dass** die Dicke (12) zwischen 0,5 und 2 mm liegt.

8. Biologische Prothese (10) nach einem der vorhergehenden Ansprüche, **dadurch gekennzeichnet, dass** die Länge (t) des Bereichs (18) zwischen 1 und 8 cm liegt.

9. Biologische Prothese (10) nach einem der vorhergehenden Ansprüche, **dadurch gekennzeichnet, dass** die Länge (6) des Bereichs (18) zwischen 1 und 4 cm liegt.

10. Biologische Prothese (10) nach einem der vorhergehenden Ansprüche, **dadurch gekennzeichnet, dass** der untere Bereich des Kegelstumpfs einen Durchmesser D zwischen 2 und 8 cm hat.

11. Biologische Prothese (10) nach einem der vorhergehenden Ansprüche, **dadurch gekennzeichnet, dass** diese gefriergetrocknet ist.

12. Verfahren zur Herstellung einer biologischen Prothese (10) nach einem der vorhergehenden Ansprüche, **dadurch gekennzeichnet, dass** dieses die folgenden Verfahrensschritte umfasst:
- Entnahme des Teguments des Tiers aus dem Teil (14) mit den gewünschten Abmessungen, wobei die Entnahme bei einem toten Tier unmittelbar nach dessen Schlachtung direkt im Schlachthof erfolgt,
- Trennen der Dermis vom Rest des Teguments,
- gegebenenfalls Zerteilen der entnommenen Dermis hinsichtlich der Dicke, um die gewünschte Dicke (12) zu erhalten,
- Behandeln der Dermis mit Dezellularisierung und Antigenisierung, und
- Sterilisierung.

13. Verfahren zur Herstellung nach einem der vorhergehenden Ansprüche, **dadurch gekennzeichnet, dass** dieses nach der Behandlung einen Verfahrensschritt der Gefriertrocknung und gegebenenfalls des Herrichtens in einer sterilen Verpackung umfasst.

## Claims

1. A biological prosthesis (10) having a thickness (12) comprising between 0.5 and 4 mm, formed by the dermis taken from a part (14) of an animal belonging to the porcine, bovine or caprine family, said part (14) of the animal corresponding to a part going from the region (16) situated at the base of the tail opposite the junction of the last sacral vertebrae as far as a region (18) starting from the base of the tail covering the circumference of at least part of the caudal vertebrae.

2. A biological prosthesis according to claim 1, **characterised in that** the dermis is a dermis treated by acellularisation and antigenisation.

3. A biological prosthesis (10) according to one of the preceding claims, **characterised in that** the region (16) situated at the base of the tail opposite the junction of the last sacral vertebrae is in the form of a truncated cone.

4. A biological prosthesis (10) according to one of the preceding claims, **characterised in that** the region (18) covering the circumference of at least some of the caudal vertebrae is cylindrical in shape.

5. A biological prosthesis (10) according to one of the preceding claims, **characterised in that** the mean diameter of the region (18) comprises between 1 and 4 cm.

6. A biological prosthesis (10) according to one of the preceding claims, **characterised in that** the mean diameter (d) of the region (18) comprises between 1.5 and 3 cm.

7. A biological prosthesis (10) according to one of the preceding claims, **characterised in that** the thickness (12) comprises between 0.5 and 2 mm.

8. A biological prosthesis (10) according to one of the preceding claims, **characterised in that** the length (t) of the region (18) comprises between 1 and 8 cm.

9. A biological prosthesis (10) according to one of the preceding claims, **characterised in that** the length (t) of the region (18) comprises between 1 and 4 cm.

10. A biological prosthesis (10) according to one of the preceding claims, **characterised in that** the lower part of the truncated cone has a diameter (D) comprising between 2 and 8 cm.

11. A biological prosthesis (10) according to one of the preceding claims, **characterised in that** it is lyophilised.

12. A method for manufacturing a biological prosthesis (10) according to one of the preceding claims, **characterised in that** it comprises the following steps:
- taking the tegument of the animal over the part (14) to the required dimensions, the taking being done on a dead animal, just after it has been slaughtered, directly in the abattoir,
- separating the dermis from the rest of the tegument,
- optionally splitting the dermis taken in the thickness, in order to obtain the required thickness (12),
- treatment of the dermis by acellularisation and antigenisation,
- sterilisation.

13. A manufacturing method according to the preceding claim, **characterised in that** it comprises, after treatment, a step of lyophilisation and optionally packaging in a sterile package.
